# FASCICULE DE BREVET EUROPEEN

(11) **EP 0 672 139 B1**
(45) Date de publication et mention de la délivrance du brevet: **04.05.2005**
(21) Numéro de dépôt: 93924683.1
(22) Date de dépôt: 08.11.1993
(51) Int. Cl.: C12N 15/12, C12Q 1/68, G01N 33/68, C12N 5/10, C12N 1/21

(54) **NOUVEAUX POLYPEPTIDES AYANT UNE ACTIVITE DE RECEPTEUR OPIOIDE, ACIDES NUCLEIQUES CODANT POUR CES POLYPEPTIDES ET UTILISATIONS**
POLYPEPTIDE MIT OPIOIDER REZEPTOR-AKTVITÄT, FÜR SIE KODIERENDE NUKLEINSÄUREN UND IHRE VERWENDUNGEN
NOVEL POLYPEPTIDES HAVING OPIOID RECEPTOR ACTIVITY, NUCLEIC ACIDS CODING THEREFOR AND USES THEREOF

(30) Priorité: 10.11.1992 FR 9213526
(43) Date de publication de la demande: 20.09.1995
(73) Titulaire: AstraZeneca AB, 151 85 Södertälje (SE)
(72) Inventeur: KIEFFER, Brigitte, F-67000 Strasbourg (FR)
(86) Numéro de dépôt international: PCT/FR1993/001097
(87) Numéro de publication internationale: WO 1994/011500

(56) Documents cités:
- WO-A-90/00564
- WO-A-91/18901
- JOURNAL OF CHROMATOGRAPHY vol. 597, no. 1-2 , 24 Avril 1992 , AMSTERDAM NL pages 429 - 433 Fujioka T;Inoue F;Sumita S;Kuriyama M;Hanawa T;Katagi T; 'Purification and reconstitution of mu-opioid receptors in liposome.'
- PROCEEDINGS OF THE NATIONAL ACADEMY OF SCIENCES OF USA. vol. 89 , Mai 1992 , WASHINGTON US pages 4124 - 4128 XIE, G. ET AL; 'Expression cloning of cDNA encoding a seven-helix receptor from human placenta with affinity for opiod ligands' cité dans la demande
- PATENT ABSTRACTS OF JAPAN vol. 16, no. 338 (C-965) 22 Juillet 1992 & JP,A,04 099 797 (SNOW BRAND MILK PROD. CO. LTD) 31 Mars 1992
- PROCEEDINGS OF THE NATIONAL ACADEMY OF SCIENCES OF USA. vol. 89 , Décembre 1992 , WASHINGTON US pages 12048 - 12052 KIEFFER, B.L. ET AL.; 'The delta-opioid receptor : Isolation of a cDNA by expression cloning and pharmacological characterization'
- PROCEEDINGS OF THE NATIONAL ACADEMY OF SCIENCES OF USA. vol. 90 , Juillet 1993 , WASHINGTON US pages 6736 - 6740 YASUDA, K. ET AL.; 'Cloning an functional comparison of kappa and delta opiod receptors from mouse brain'
- TRENDS IN PHARMACOLOGICAL SCIENCES vol. 13 , Mai 1992 , CAMBRIDGE GB pages 185 - 193 DI CHIARA, G. ET AL.; 'Neurobiology of opiate abuse'
- JOURNAL OF RECEPTOR RESEARCH vol. 7, no. 1-4 , 1987 pages 105 - 132 SIMON, E.J. ET AL.; 'Subunit structure and purification of opiod receptors'

## Description

La présente invention concerne de nouveaux polypeptides et le matériel génétique permettant leur expression. Plus particulièrement, elle concerne de nouveaux polypeptides ayant une activité de récepteur opioïde.

Les récepteurs opioïdes sont connus depuis longtemps comme des récepteurs membranaires du système nerveux, médiant les effets analgésiques des alkaloïdes dérivés de l'opium. Des ligands endogènes de ces récepteurs et leurs précurseurs ont été caractérisés, et leur role dans la réponse à la douleur et au stress a été largement étudié (Akil et al., (1984) Annu. Rev. Neurosci. 7, 223-255). Par ailleurs, des études pharmacologiques ont révélé l'existence de trois sous-types de récepteurs opioïdes : les récepteurs mu (morphine), delta (enképhaline) et kappa (dynorphine). Ces mêmes études ont également montré que l'action inhibitrice de l'activité cellulaire de ces récepteurs était liée à l'activation de protéines G (Simonds, W.F. (1988) Endocrine Rev. 9, 200-212). Pour cette raison, ces récepteurs sont aujourd'hui classés dans la famille des récepteurs qui interagissent avec des protéines G, une classe de récepteurs possèdant sept domaines transmembranaires et englobant environ 80 % des récepteurs connus.

Différents laboratoires ont essayé de cloner des gènes codant pour des récepteurs opioïdes. Notamment, une protéine liant les opioïdes avec une sélectivité de type mu a été purifiée à partir de cerveau de boeuf et partiellement séquencée. Un ADNc a ensuite été isolé en utilisant des sondes nucléotidiques dérivées de cette séquence partielle. Cependant, la protéine déduite de cette séquence ne possède pas de domaine transmembranaire et présente une forte homologie avec la NCAM, une molécule d'adhésion (Schofield et al., (1989) EMBO J. 8,489-495). Plus récemment, l'isolement d'un autre ADNc a été décrit (Xie et al., (1992) Proc. Natl. Acad. Sci. USA 89, 4124- 4128), obtenu par clonage par expression. La banque d'ADNc était construite à partir de placenta humain, exprimant exclusivement le sous-type kappa, et elle a été criblée au moyen d'un peptide dérivé de la dynorphine par une technique d'enrichissement par affinité. Cependant, cette protéine possède une affinité relativement faible pour les ligands opioïdes, ne présente aucune spécificité de sous-type, et semble enfin très similaire au récepteur à la neuromédine K. Enfin, tous les essais de clonage de récepteurs opioïdes par PCR basés sur les homologies avec les récepteurs couplés aux protéines G se sont révélés infructueux.

La présente invention décrit pour la première fois le clonage de gènes codant pour des récepteurs opioïdes. La présente invention décrit également pour la première fois la séquence de récepteurs opioïdes et leur expression dans des cellules recombinantes. La présente invention permet ainsi une meilleure compréhension de la structure des récepteurs opioïdes, et d'étudier de manière plus fine leur mécanisme d'action. La présente invention permet également d'obtenir des récepteurs opioïdes de très grande pureté et en quantité élevée, permettant la réalisation d'études fonctionnelles et pharmacologiques, la fabrication d'anticorps, etc. L'invention permet aussi de préparer des fragments de récepteurs opioïdes de taille définie, ainsi que toutes sortes de dérivés des récepteurs opioïdes. L'invention fournit également des cellules recombinantes exprimant des récepteurs opioïdes ou des fragments de récepteurs opioïdes, utilisables pour le criblages de ligands de ces récepteurs (agonistes, antagonistes, modulateurs, etc). Les séquences d'ADN de l'invention permettent également la réalisation de sondes, capables de détecter dans des échantillons biologiques toute irrégularité dans l'expression d'un récepteur opioïde (non-expression, mutation, polymorphisme, etc). Ces sondes sont également utilisables pour le clonage par hybridation de tout autre ADNE codant pour un récepteur opioïde, à partir de tissus d'origines diverses et notamment d'origine humaine, comme indiqué plus loin.

Un premier objet de l'invention réside donc dans une séquence nucléotidique codant pour un polypeptide ayant une activité de récepteur opioïde. Au sens de l'invention, récepteur opioïde comprend notamment les sous-types delta, mu et kappa.

Préférentiellement, l'invention a pour objet une séquence nucléotidique codant pour un polypeptide ayant une activité de récepteur opioïde delta.

Encore plus préférentiellement, la séquence nucléotidique selon l'invention est choisie parmi :
(a) tout ou partie de la séquence nucléotidique SEQ ID n°1 ou de son brin complémentaire,
(b) toute séquence hybridant avec une séquence (a) et codant pour un polypeptide ayant une activité de récepteur opioïde, et,
(c) les séquences dérivées des séquences (a) et (b) en raison de la dégénérescence du code génétique.

Un mode de réalisation tout particulier de l'invention est représenté par une séquence nucléotidique comprenant tout ou partie de la séquence nucléotidique SEQ ID n°1 ou de son brin complémentaire.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base de la séquence SEQ ID n°1. De telles banques peuvent être préparées à partir de cellules de différentes origines par des techniques classiques de biologie moléculaire connues de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatiqe de séquences obtenues par criblage de banques.

Les séquences nucléotidiques de l'invention peuvent être utilisées pour la production des polypeptides opioïdes. Le terme polypeptide opioïde désigne tout polypeptide ayant une activité de récepteur opioïde, et tout fragment ou dérivé d'un tel polypeptide. Pour la production de polypeptides opioïdes, la partie codant pour ledit polypeptide est généralement placée sous le contrôle de signaux permettant son expression dans un hôte cellulaire. Le choix de ces signaux (promoteurs, terminateurs, etc) peut varier en fonction de l'hôte cellulaire utilisé. A cet effet, les séquences nucléotidiques de l'invention peuvent faire partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Les hôtes cellulaires utilisables pour la production des polypeptides opioïdes de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula*. S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme hôtes procaryotes, on préfère utiliser les bactéries suivantes *E.coli, Bacillus*, ou *Streptomyces*.

Les séquences nucléotidiques de la présente invention sont également utilisables dans le domaine pharmaceutique, soit pour la réalisation de séquences antisens utilisables dans le cadre d'une thérapie génique, soit encore pour la réalisation de sondes permettant la détection, par des expériences d'hybridation, de l'expression de récepteurs opioïdes dans des échantillons biologiques et la mise en évidence d'anomalies génétiques (polymorphisme, mutations) ou d'expressions aberrantes.

L'inhibition de l'expression de certains gènes par des séquences antisens s'est avérée être une stratégie prometteuse dans le contrôle de l'activité d'un gène. Les séquences antisens sont des séquences dont le produit de transcription est complémentaire du brin codant d'un gène donné, et est de ce fait capable d'hybrider spécifiquement avec l'ARNm transcrit, inhibant sa traduction en protéine. L'invention a ainsi pour objet les séquences antisens capables d'inhiber au moins partiellement la production de polypeptides opioïdes tels que définis précédemment. De telles séquences peuvent être constituées par tout ou partie des séquences nucléotidiques définies ci-avant. Il s'agit généralement de séquences ou de fragments de séquences complémentaires de séquences codant pour des peptides de l'invention. De telles séquences peuvent être obtenues à partir de la séquence SEQ ID n°1, par fragmentation, etc, ou par synthèse chimique.

Comme indiqué ci-dessus, l'invention permet également la réalisation de sondes nucléotidiques, synthétiques ou non, capables de s'hydrider avec les séquences nucléotidiques définies ci-avant qui codent pour des polypeptides opioïdes de l'invention, ou avec les ARNm correspondants. De telles sondes peuvent être utilisées *in vitro* comme outil de diagnostic, pour la détection de l'expression d'un récepteur opioïde, ou encore pour la mise en évidence d'anomalies génétiques (mauvais épissage, polymorphisme, mutations ponctuelles, etc). Compte tenu des activités multiples des ligands endogènes des récepteurs opioïdes, les sondes de l'invention peuvent ainsi permettre d'identifier des affections neurologique, cardiovasculaire ou psychiatrique. Ces sondes peuvent également être utilisées pour la mise en évidence et l'isolement de séquences d'acides nucléiques homologues codant pour des polypeptides opioïdes tels que définis précédemment, à partir d'autres sources cellulaires et préférentiellement de cellules d'origines humaines. Bien que la présente demande soit plus particulièrement illustrée par un récepteur de type delta, les études biochimiques et immunologiques décrites dans la littérature indiquent clairement que les récepteurs opioïdes possèdent des homologies importantes (même poids moléculaires, réactivité croisée avec les mêmes anticorps, etc). Les sondes de l'invention comportent généralement au moins 10 bases, et elles peuvent comporter jusqu'à l'intégralité de la séquence SEQ ID n°1 ou de son brin complémentaire. Préférentiellement, ces sondes sont, préalablement à leur utilisation, marquées. Pour cela, différentes techniques connues de l'homme du métier peuvent être employées (marquage radioactif, enzymatique, etc). Les conditions d'hybridation dans lesquelles ces sondes peuvent être utilisées sont indiquées dans les techniques générales de clonage ci-après ainsi que dans les exemples.

L'invention a également pour objet tout polypeptide résultant de l'expression d'une séquence nucléotidique telle que définie précédemment. Préférentiellement, il s'agit d'un polypeptide comprenant tout ou partie de la séquence peptidique SEQ ID n° 2 ou d'un dérivé de celle-ci.

Au sens de la présente invention, le terme dérivé désigne toute molécule obtenue par modification de nature génétique et/ou chimique de la séquence peptidique SEQ ID n° 2. Par modification de nature génétique et/ou chimique, on peut entendre toute mutation, substitution, délétion, addition et/ou modification d'un ou plusieurs résidus. De tels dérivés peuvent être générés dans des buts différents, tels que notamment celui d'augmenter l'affinité du peptide pour son(ses) ligand(s), celui d'améliorer ses niveaux de production, celui d'augmenter sa résistance à des protéases, celui d'augmenter et/ou de modifier son activité, ou celui de lui conférer de nouvelles propriétés pharmacocinétiques et/ou biologiques. Parmi les dérivés résultant d'une addition, on peut citer par exemple les polypeptides chimères comportant une partie hétérologue supplémentaire liée à une extrêmité. Le terme dérivé comprend également les polypeptides homologues au polypeptide SEQ ID n° 2, issus d'autres sources cellulaires et notamment de cellules d'origine humaine, ou d'autres organismes, et possédant une activité de même type. De tels polypeptides homologues peuvent être obtenus par des expériences d'hybridation comme décrit dans les exemples.

Préférentiellement, les polypeptides de l'invention sont des polypeptides possédant la capacité de lier la morphine (récepteur type mu), l'enképhaline (récepteur type delta) ou la dynorphine (récepteurs type kappa). Encore plus préférentiellement, il s'agit de polypeptides possédant la capacité de lier l'enképhaline (récepteur type delta). Toujours selon un mode préféré, les polypeptides de l'invention sont susceptibles d'être reconnus par des anticorps reconnaissant la séquence peptidique SEQ ID n° 2 complète. De tels anticorps peuvent être générés par toute technique connue de l'homme du métier, en utilisant comme antigènes les polypeptides décrits dans la présente demande.

Comme indiqué dans les exemples, ces polypeptides peuvent être exprimés dans différents types cellulaires pour former des récepteurs opioïdes fonctionnels.

Les polypeptides de l'invention peuvent être obtenus par expression dans un hôte cellulaire d'une séquence nucléotidique telle que décrite ci-dessus, par synthèse chimique, sur la base de la séquence SEQ ID n° 2 en utilisant les techniques connues de l'homme du métier, ou par une combinaison de ces techniques.

Un autre objet de l'invention concerne les cellules recombinées capables d'exprimer à leur surface un polypeptide ayant une activité de récepteur opioïde. Ces cellules peuvent être obtenues par introduction d'une séquence nucléotidique telle que définie ci-dessus, puis culture desdites cellules dans des conditions d'expression de ladite séquence.

Les cellules recombinées selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces*, ou *Hansenula*. S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme cellules procaryotes, on préfère utiliser les bactéries suivantes *E.coli, Bacillus*, ou *Streptomyces.* Les cellules ainsi obtenues peuvent être utilisées pour mesurer la capacité de différentes molécules à se comporter comme ligand ou comme modulateur de l'activité des récepteurs opioïdes. Plus particulièrement, elles peuvent ainsi être utilisées dans un procédé de mise en évidence et d'isolement de ligands ou de modulateur de l'activité des récepteurs opioïdes, et, plus préférentiellement, d'agonistes et d'antagonistes.

Un autre objet de l'invention concerne donc un procédé de mise en évidence et/ou d'isolement de ligands des récepteurs opioïdes, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur opioide dans des conditions permettant l'interaction entre ledit polypeptide et ladite molécule dans le cas où celle-ci posséderait une affinité pour ledit polypeptide, et,
- on détecte et/ou isole les molécules liées au dit polypeptide.

Dans un mode particulier, ce procédé de l'invention est adapté à la mise en évidence et/ou l'isolement d'agonistes et d'antagonistes de l'enképhaline pour les récepteurs opioïdes delta.

Un autre objet de l'invention concerne un procédé de mise en évidence et/ou d'isolement de modulateurs des récepteurs opioïdes, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur opioïde, en présence du ligand endogène dudit récepteur, dans des conditions permettant l'interaction entre ledit polypeptide et son ligand, et,
- on détecte et/ou isole les molécules capables de moduler l'activité du ligand sur ledit polypeptide.

Dans un mode particulier, ce procédé de l'invention est adapté à la mise en évidence et/ou l'isolement de modulateurs de l'activité de l'enképhaline sur les récepteurs opioïdes delta.

Un autre objet de l'invention concerne l'utilisation d'un ligand ou d'un modulateur identifié et/ou obtenu selon le procédé décrit ci-avant comme médicament. De tels ligands ou modulateurs peuvent en effet permettre de traiter certaines affections liées aux récepteurs opioïdes. En particulier, les récepteurs opioïdes étant les médiateurs d'un effet analgésique, les agonistes de ces récepteurs peuvent être utilisés pour diminuer les sensations de douleur. Par ailleurs, ces récepteurs étant les médiateurs des effets des dérivés de l'opium, leurs antagonistes peuvent être utilisés dans le cadre d'un traitement de désintoxication.

L'invention concerne également tout médicament comprenant comme principe actif au moins une molécule agissant sur un récepteur de l'invention. Préférentiellement la molécule est un ligand ou un modulateur identifié et/ou isolé selon le procédé décrit précédemment.

D'autres avantages de la présente invention apparaîtront à la lecture des exemples qui suivent, qui doivent être considérés comme illustratifs et non limitatifs.

### Légende des figures

Figure 1 : Courbe de liaison du ³H-DTLET aux membranes des cellules Cos-1 exprimant le récepteur K56. Les membranes ont été incubées avec des concentrations croissantes de ligand, avec ou sans 1 µM de naloxone (-). La liaison spécifique est représentée. L'encart représente l'analyse en Scatchard des résultats.
Figure 2 : Courbe de liaison du ³H-DTLET aux membranes des cellules Cos-1 exprimant le récepteur K56, en présence de concentrations croissantes de compétiteurs.
Figure 3 : Courbe de liaison du ³H-DTLET (1 nM) aux membranes des cellules Cos-1 exprimant le récepteur K56, en présence de concentrations croissantes de compétiteurs.
Table 1 : Profil pharmacologique du récepteur K56. Les résultats correspondent à des expériences de compétition pour la liaison du ³H-DTLET aux membranes des cellules Cos-1 exprimant le récepteur K56 de manière transitoire. Les valeurs d'IC50 (correspondant à la concentration en ligand nécessaire pour déplacer 50 % du ³H-DTLET lié) ont été calculées expérimentalement et converties en Ki selon l'équation suivante : Ki = IC50/(1 + L/Kd) dans laquelle L est la concentration en ³H-DTLET et Kd est la constante de dissociation du ³H-DTLET. Chaque valeur correspond à la moyenne de 3 expériences indépendantes.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli*, etc, sont bien connues de l'homme de métier et sont abondament décrites dans la littérature [Maniatis T. et al., "Molecular Cloning, a Laboratory Manual". Cold Spring Harbor Laboratory, Cold Spring Harbor, N.Y., 1982; Ausubel F.M. et al. (eds), "Current Protocols in Molecular Biology", John Wiley & Sons, New York, 1987].

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 selon les recommandations du fournisseur.

Le remplissage des extrémités 5' proéminentes est effectué par le fragment de Klenow de l'ADN Polymérase I d'*E.coli* selon les spécifications du fournisseur. La destruction des extrémités 3' proéminentes est effectuée en présence de l'ADN Polymérase du phage T4 utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764].

L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350].

La vérification des séquences nucléotidiques est effectuée par la méthode développée par Sanger et al. [Proc. Natl. Acad. Sci. USA, 74 (1977) 5463-5467].

Pour les expériences d'hybridation, les conditions de stringence sont basées sur Maniatis T. et al., précitée.

### 1. Isolement du récepteur opioïde delta de souris

Cet exemple décrit l'isolement du clone K56 codant pour le récepteur opioïde delta de souris par criblage d'une banque d'ADNc par clonage par expression.

### 1.1. Construction de la banque

Une banque d'ADNc a été réalisée à partir de l'hybridome NG108-15 (Klee, W.A., et Nirenberg, M. (1974) Proc. Natl. Acad. Sci. USA 71, 3474-3477) en utilisant le vecteur d'expression transitoire mammifère pCDM8 (Aruffo, A. et Seed, B. (1987) Ploc. Natl. Acad Sci. USA 84, 8573-8577. Pour cela, les cellules NG108-15 [fournies par B. Foucaud (URA 1836, Faculté de Pharmacie, Strasbourg, France)] ont été récoltées à 50 % de la confluence, l'ARN a été extrait de ces cellules par précipitation en présence de chlorure de lithium et d'urée (Auffray et al (1980) Eur. J. Biochem. 107, 303-314), et les ARN polyA ont été purifiés sur colonne d'oligo(dT) (Pharmacia). La banque d'ADNc a ensuite été construite chez E.coli dans le vecteur pCDM8 (fournit par B. Seed, Massachusetts General Hospital, USA) selon la technique décrite par Kieffer (1991, Gene 109, 115-119). Environ 300 000 transformants ont été cultivés une nuit dans des boîtes de pétri (16 cm) à une densité de 3000 colonies par boîte. Les colonies ont ensuite été transférées en milieu sélectif LB. La moitié de la suspension obtenue a été conservée sous forme congelée à -80°C en présence de glycérol 30 %, et le reste a été utilisé pour préparer l'ADN plasmidique, selon la technique de lyse alkaline (Sambrook, J., Fritsch, E.F. & Maniatis, T. (1989) Molecular Cloning : A Laboratory Manual (Cold Spring Harbor Lab., Cold Spring Harbor, NY), 2nd Ed.).

Un dizième de l'ADN plasmidique de chaque lot a été transfecté indépendemment dans les cellules Cos-1 (ATCC CRL/1650) en utilisant la technique au DEAE-dextran. La meilleure efficacité de transfection (jusqu'à 20 % de cellules transfectées) a été obtenue en suivant le protocole suivant : Les cellules Cos-1 monocouches ont été cultivées dans des boîtes de 6 cm à une densité de 2.10⁵ cellules par boîte, dans du milieu DMEM (Dulbecco Modified Eagle Medium) en présence de 10 % de sérum de veau foetal (FCS) et sous 5 % de CO₂. Après 16 heures à 37°C, les cellules ont été lavées 2 fois dans un tampon phosphate (PBS, pH 7,4) et incubées 1 heure à 37°C avec une solution comprenant 1-10 µg d'ADN, 0,25 mg/ml de DEAE-dextran (Pharmacia), 10 mM de Tris-HCl, pH 7,4 dans 2 ml de DMEM. Les cellules ont ensuite été traitées avec 10 % de glycérol dans 2 ml de DMEM pendant 3 minutes, rapidement diluées avec 5 ml de PBS et lavées 2 fois avec du PBS. Finalement, les cellules ont été incubées 5 heures à 37°C dans du milieu DMEM contenant 10 % de FCS et 0,1 mM de chloroquine, lavées 1 fois dans du milieu DMEM contenant 10 % de FCS puis cultivées pendant 72 heures dans ce milieu.

### 1.2. Criblage par expression

Les cellules Cos-1 transfectées décrites en 1.1. ci-dessus ont été testées pour leur capacité à lier le peptide suivant : Tyr-D-Thr-Gly-Phe-Leu-Thr (DTLET) marqué au tritium (61 Ci/mmole, CEA, Saclay, France). L'utilisation d'un ligand ayant une activité spécifique faible dans un tel test de criblage n'avait encore jamais été décrite. Les cellules en monocouche ont été lavées 2 fois en présence de PBS supplémenté de 0,5 % de sérum-albumine de boeuf (BSA), et incubées 35 minutes à 37°C en présence de PBS supplémenté de 0,5 % de BSA, contenant 1 nM ³H DTLET (2ml), qui correspond au Kd des récepteurs natifs. Les boîtes ont été refroidies sur la glace pendant 5 minutes et les cellules lavées 4 fois avec du PBS supplémenté de 0,5 % de BSA glacé. Les cellules ont ensuite été solubilisées dans 1,5 ml de SDS 1 %, ajoutées à 7 ml de liquide de scintillation et comptées (LS6000SC, Beckman).

Le fractionnement des lots positifs a été réalisé en diluant un aliquot du stock, congelé dans le glycérol, dans un milieu sélectif, puis en étalant la suspension sur des membranes de nitrocellulose déposées sur des plaques d'agar. Après culture une nuit, des fragments de membranes ont été découpés afin d'isoler les lots de colonies indépendantes. Ces différentes colonies ont été transférées dans 10 ml de milieu sélectif LB et cultivées jusqu'à une DO₆₀₀nm de 0,5. Un aliquot a été congelé dans le glycérol et le reste a été utilisé pour préparer l'ADN plasmidique et transfecter les cellules Cos-1, comme décrit ci-avant.

Un premier lot positif a été détecté, qui produisait un signal dépassant le bruit de fond de 10 %. Ce lot a été divisé en 40 lots de 250 clones. Ces lots ont été testés à nouveau pour leur capacité de lier le ³H-DTLET. Pour évaluer la spécificité du ³H-DTLET lié, les clones ainsi fractionnés ont été incubés en présence de naloxone froid, un antagoniste des opioides. Plusieurs clones produisent un signal dépassant le bruit de fond de 20 % et disparaissant en présence de naloxone. L'un d'entre eux a été sous-divisé 2 fois (40 lots de 30 clones puis 90 clones individuels), conduisant à un clone unique, désigné K56, conférant aux cellules Cos-1 une forte activité de liaison du ³H-DTLET. Notamment, le signal obtenu est 6 fois supérieur au bruit de fond, sensible au naloxone et dépendant de la concentration en ³H-DTLET.

### 2. Etude structurale du Récepteur Opioïde delta porté par le clone K56

Le clone K56 contient un insert de 2,2 kb environ, dont la séquence a été déterminée sur les 2 brins, en utilisant la technique des didéoxynucléotides (kit sequenase, US Biochemicals).

La séquence ainsi obtenue correspond à la séquence SEQ ID n° 1. Elle montre que l'ADNc isolé porte une phase de lecture ouverte de 1174 pb codant pour une protéine de 371 acides aminés (SEQ ID n° 2), de poids moléculaire calculé de 40810 daltons (le site d'initiation de la traduction a été attribué au codon ATG en position 59 sur cette ORF). Par ailleurs, l'analyse d'hydrophobicité montre que cette protéine porte 7 domaines hydrophobes transmembranaires (Tm), une particularité rencontrée chez les membres de la famille des récepteurs couplés à des protéines G. De plus, les résidus conservés chez ces protéines sont également présents dans la protéine K56, à savoir :
- une proline dans les domaines Tm IV, V, VI et VII,
- un tryptophane dans les domaines Tm IV et VI et dans la première boucle extracellulaire,
- les séquences consensus GNxxV (Tm I) ; LAxAD (Tm II) ; DRY (2ème boucle intracellulaire ; et NPxxY (Tm VII)

Par ailleurs, la protéine K56 possède un résidu Asp en position 128 qui est équivalent au résidu Asp 113 des récepteurs β-adrénergiques et qui pourrait donc être impliquée dans le site de liaison des ligands.

La séquence de la protéine K56 a finalement été comparée avec les séquences d'autres protéines de la famille des récepteurs couplés à des protéines G, à savoir :
- le récepteur β2-adrénergique,
- le récepteur de la neuromédine K,
- le récepteur de la rhodopsine, et
- le récepteur du peptide N-formyl.

L'homologie la plus importante obtenue est de 30 %, incluant 10 gaps. C'est l'homologie la plus forte obtenue avec les protéines de la banque Swissprot Data.

### 3. Etude pharmacologique du récepteur K56

Le plasmide K56 isolé dans l'exemple 1 a été utilisé pour transfecter des cellules Cos-1. Les membranes des cellules transfectées obtenues ont ensuite été préparées et testées pour leur capacité à lier certains ligands opioïdes marqués.

Le plasmide K56 purifié sur chlorure de césium (plasmide pCDM8 contenant l'insert de 2,2 kb environ codant pour le récepteur opioïde) a été utilisé pour transfecter les cellules Cos-1 (sur plaque de 10 cm) en utilisant la technique au DEAE-dextran. Le témoin est constitué par les cellules transfectées par le plasmide pCDM8.

72 heures après la transfection, les cellules recombinantes sont récoltées et les membranes sont préparées de la manière suivante : les culots cellulaires sont repris, à 4°C, dans 60 ml de tampon Tris-HCl 50 mM pH 7,4; EDTA 10 mM, homogénéisés et centrifugés à 1100 g pendant 10 minutes. Le culot est ensuite repris une seconde fois dans 30 ml du même tampon, homogénéisé et centrifugé. Les 2 surnageants sont réunis et centrifugés à 110 000 g pendant 15 minutes. Le culot membranaire est alors repris dans 5ml du même tampon, aliquoté et conservé à -80°C. Des expériences de liaison à saturation et de compétition ont ensuite été réalisées sur ces membranes en présence de différents ligands. Pour cela, les échantillons de membrane (15-30 µg de protéines) ont été incubés 30 minutes à 37°C en présence de ³H-DTLET, avec ou sans compétiteur, dans un volume final de 1 ml de tampon Tris-HCl 50 mM (pH 7,4); EDTA 10 mM. La réaction est ensuite stopée par filtration sous vide sur filtres Whatman GF/B, et rinçage 3 fois avec 3 ml du tampon froid. Les valeurs de Ki ont été obtenues en suivant l'équation de Cheng et Prussof : Ki = IC50 / (1 + L/Kd). La radioactivité a été mesurée avec un compteur β.

### 3.1. Affinité du DTLET pour les cellules Cos-1 transfectées.

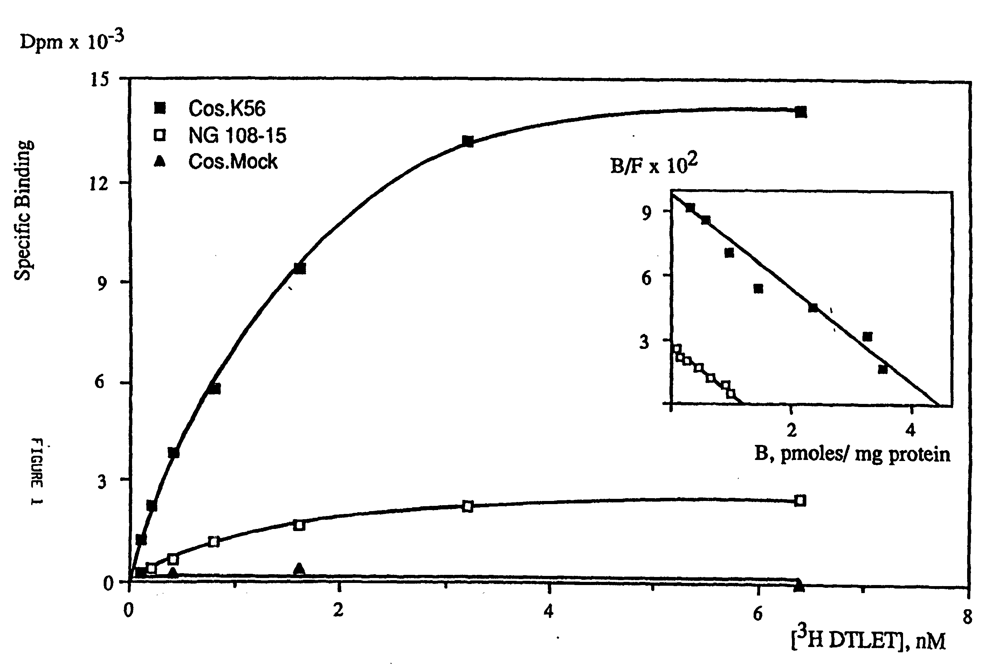

L'affinité du DTLET pour les cellules Cos-1 transfectées a été déterminée dans les conditions opératoires générales décrites ci-dessus. Les membranes ont été incubées en présence de concentrations croissantes de ³H-DTLET, en l'absence (liaison totale) ou en présence (liaison non spécifique) de Naloxone (-) à 10⁻⁶ M. La liaison spécifique correspond à la différence entre liaison totale et liaison non spécifique. Les résultats obtenus montrent qu'une liaison spécifique élevée est observée pour les membranes des cellules Cos-1 transfectées par le plasmide K56, alors qu'elle est négligeable pour les membranes des cellules témoin (cellules Cos-1 transfectées par le plasmide pCDM8) (voir figure 1). La liaison aux membranes des cellules NG 108-15 qui expriment naturellement le récepteur opioïde delta est présentée à titre comparatif.

L'analyse en Scatchard de la liaison spécifique montre la présence d'une seule classe de récepteurs, avec une constante de dissociation apparente Kd = 1,4 nM; et un Bmax compris entre 3,9 et 6,4 pmoles par mg de protéines selon les préparations membranaires (figure 1). Compte tenu de l'efficacité de transfection (environ 10 %), le niveau d'expression des polypeptides opioïdes de l'invention dans les cellules Cos-1 transfectées peut être estimé à 5.10⁶ molécules / cellules.

### 3.2. Expériences de compétition.

Pour les expériences de compétition, le ³H-DTLET a été utilisé à une concentration de 1 nM, en présence de concentrations croissantes de compétiteurs froids. Les compétiteurs froids utilisés sont les suivants :
- DADLE : [D-Ala², D-Leu⁵]-enképhaline, Sigma
- DPDPE : [D-penicillamine², D-penicillamine⁵]-enképhaline cyclique, Sigma
- DAGO : [D-Ala², MePhe⁴, Gly-ol⁵]-enképhaline, Sigma
- U50488 : trans-(±)-3,4-dichloro-N-méthyl-N-(2-[1-pyrrolidinyl] cyclohexyl) benzeneacetamide, Sigma
- Naloxone (+) et (-),
- levorphanol, dextrorphan,
- bremazocine
- etonitazen

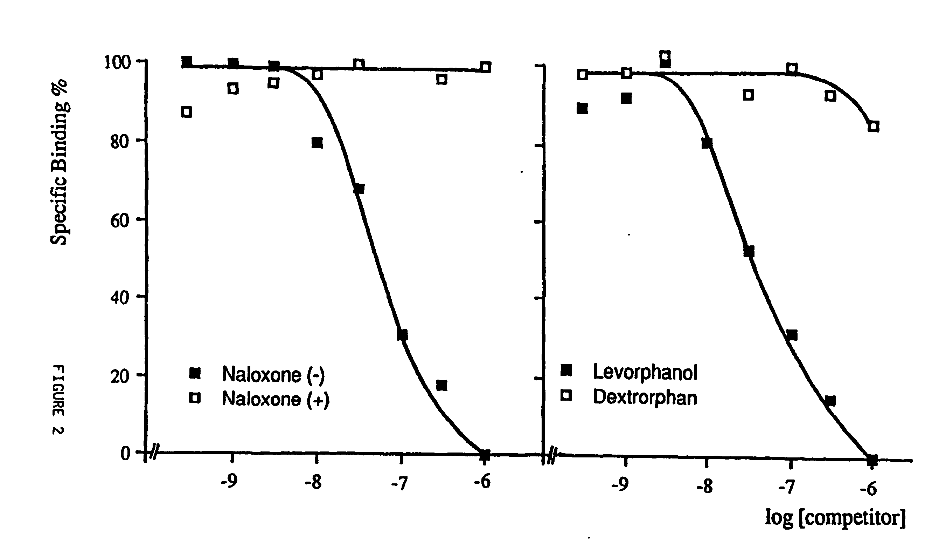

Les récepteurs opioïdes sont très stéréosélectifs. Le levorphanol et le Naloxone (-) sont connus pour lier les récepteurs opioïdes avec une haute affinité, tant dis que les énantiomères dextrorphan et naloxone (+) respectivement, ne se lient pas, indépendemment du sous-type de récepteur étudié. Des expériences de compétition conduites avec le DTLET en présence des 2 paires de ligands montrent que (i) le naloxone (+) n'est pas un compétiteur, (ii) 1 µM dextrorphan induit une légère inhibition, ce qui est en accord avec les données sur ce produit qui indiquent qu'il peut lier les récepteurs opioïdes, mais avec une affinité 1000 fois inférieure que le levorphanol; (iii) les 2 énantiomères (-) possèdent des constantes d'inhibition Ki correspondant à celles décrites dans la littérature pour un récepteur opioïde δ (29,5 nM pour le Naloxone (-) et 20,9 nM pour le levorphanol) (voir figure 2 et table 1).

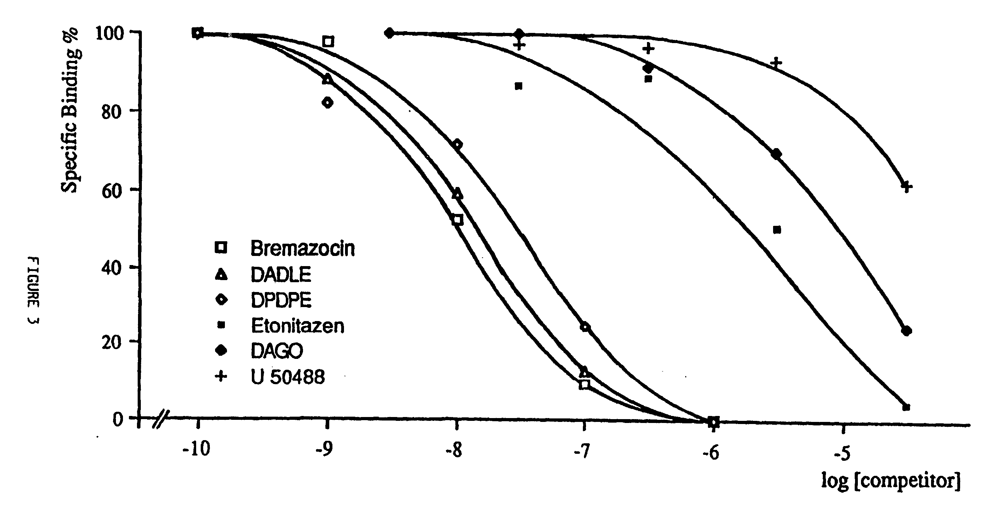

Par ailleurs, les expériences réalisées avec les autres ligands mentionnés montrent que (voir figure 3 et table 1) :
- les agonistes δ, DADLE et DPDPE, ainsi que l'agoniste non-sélectif bremazocine sont les compétiteurs les plus efficaces, avec des valeurs de Ki de 6,2; 10,9 et 5,7 nM respectivement;
- les agonistes µ, etonitazen et DAGO sont des ligands faibles, avec des valeurs de Ki de 1800 et 5050 nM respectivement; et
- l'agoniste χ, U50488 est le compétiteur le moins efficace avec un Ki de 39100 nM.

L'ordre d'efficacité des ces différents ligands confirme la classification du récepteur K56 comme récepteur opioïde delta. Ces résultats montrent par ailleurs que les cellules Cos-1 de l'invention sont bien capables d'exprimer des polypeptides opioïdes ayant des caractéristiques de liaison comparables aux récepteurs natifs.

### 4. Recherche de séquences homologues dans d'autres tissus

La séquence nucléotidique SEQ ID n° 1 ou des fragments de celle-ci peut être utilisée pour la mise en évidence de séquences homologues à partir d'autres tissus. Pour cela, différentes techniques sont utilisables, telles que la PCR, l'hybridation *in situ*., le Northern blot, etc.

Plus particulièrement, pour la recherche par PCR, les ARN totaux de différents tissus étudiés sont préparés, en utilisant la technique décrite par Cathala et al. (DNA 2(4) (1983)). Les ARN sont ensuite soumis à une transcription inverse et à une amplification, en présence de transcriptase inverse, de polymérase Taq et des sondes dérivées de la séquence SEQ ID n° 1 appropriées. Les produits ainsi obtenus sont ensuite transférés sur filtres de nitro-cellulose et hybridés dans des conditions de stringence variables.

Les séquences homologues mises en évidence lors de ces expériences peuvent évidemment être ensuite isolées et/ou amplifiées par les techniques classiques de biologie moléculaire.

### 5. Clonage du récepteur humain

Le récepteur humain homologue du récepteur décrit dans l'exemple 1 a été cloné par criblage d'une banque génomique humaine réalisée à partir de placenta, au moyen d'une sonde correspondant à la partie codante de la séquence SEQ ID n° 1. Le criblage a été réalisé par hybridation dans des conditions de stringence élevées : 5XSSC, 5X Denhardt's, 40 % formamide, 0,1 % SDS, 0,05 % NaPPI, 100 µg/ml de sperme de saumon, 42°C. Ce criblage a permis d'isoler 2 clones contenant les séquences codant pour l'homologue humain. Le premier contenait un exon codant pour les 77 premiers acides aminés de la protéine, et le deuxième contenait 2 exons, l'un codant pour les acides aminés 78 à 194 et l'autre pour les acides aminés 195 à 372. La séquence ainsi obtenue, dont certaines positions doivent être confirmées, est représentée SEQ ID n° 3.

### LISTE DE SEQUENCES

(1) INFORMATION GENERALE:
   (i) DEPOSANT:
      (A) NOM: UNIVERSITE LOUIS PASTEUR
      (B) RUE: 11 rue Humann
      (C) VILLE: STRASBOURG
      (E) PAYS: FRANCE
      (F) CODE POSTAL: 67085
   (ii) TITRE DE L' INVENTION: Nouveaux polypeptides ayant une activite de recepteur opioide, acides nucleiques codant pour ces polypeptides et utilisations.
   (iii) NOMBRE DE SEQUENCES: 3
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 2219 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Souris
   (ix) CARACTERISTIQUE ADDITIONELLE:
      (A) NOM/CLE: CDS
      (B) EMPLACEMENT: 59..1177
      (D) AUTRES RENSEIGNEMENTS: /product= "Gene récepteur opiode delta"
   (xi) DESCRIPTION DE LA SEQUENCE: SEQ ID NO: 1:
   CTCTAAAGGC TGGGTCCCTG CGCCCAGGGC GCACGGTGGA GACGGACACG GCGGCGCC       58
   ATG GAG CTG GTG CCC TCT GCC CGT GCG GAG CTG CAG TCC TCG CCC CTC     106
   Met Glu Leu Val Pro Ser Ala Arg Ala Glu Leu Gln Ser Ser Pro Leu
    1               5                  10                  15
   GTC AAC CTC TCG GAC GCC TTT CCC AGC GCC TTC CCC AGC GCG GGC GCC     154
   Val Asn Leu Ser Asp Ala Phe Pro Ser Ala Phe Pro Ser Ala Gly Ala
               20                  25                  30
   AAT GCG TCG GGG TCG CCG GGA GCC CGT AGT GCC TCG TCC CTC GCC CTA     202
   Asn Ala Ser Gly Ser Pro Gly Ala Arg Ser Ala Ser Ser Leu Ala Leu
                35                  40                  45
   GCC ATC GCC ATC ACC GCG CTC TAC TCG GCT GTG TGC GCA GTG GGG CTT     250
   Ala Ile Ala Ile Thr Ala Leu Tyr Ser Ala Val Cys Ala Val Gly Leu
       50                  55                  60
   CTG GGC AAC GTG CTC GTC ATG TTT GGC ATC GTC CGG TAC ACC AAA TTG     298
   Leu Gly Asn Val Leu Val Met Phe Gly Ile Val Arg Tyr Thr Lys Leu
   65                  70                  75                  80
   AAG ACC GCC ACC AAC ATC TAC ATC TTC AAT CTG GCT TTG GCT GAT GCG     346
   Lys Thr Ala Thr Asn Ile Tyr Ile Phe Asn Leu Ala Leu Ala Asp Ala
                   85                  90                  95
   CTG GCC ACC AGC ACG CTG CCC TTC CAG AGC GCC AAG TAC TTG ATG GAA     394
   Leu Ala Thr Ser Thr Leu Pro Phe Gln Ser Ala Lys Tyr Leu Met Glu
               100                 105                 110
   ACG TGG CCG TTT GGC GAG CTG CTG TGC AAG GCT GTG CTC TCC ATT GAC     442
   Thr Trp Pro Phe Gly Glu Leu Leu Cys Lys Ala Val Leu Ser Ile Asp
           115                 120                 125
   TAC TAC AAC ATG TTC ACT AGC ATC TTC ACC CTC ACC ATG ATG AGC GTG     490
   Tyr Tyr Asn Met Phe Thr Ser Ile Phe Thr Leu Thr Met Met Ser Val
       130                 135                 140
   GAC CGC TAC ATT GCT GTC TGC CAT CCT GTC AAA GCC CTG GAC TTC CGG     538
   Asp Arg Tyr Ile Ala Val Cys His Pro Val Lys Ala Leu Asp Phe Arg
   145                 150                 155                 160
   ACA CCA GCC AAG GCC AAG CTG ATC AAT ATA TGC ATC TGG GTC TTG GCT     586
   Thr Pro Ala Lys Ala Lys Leu Ile Asn Ile Cys Ile Trp Val Leu Ala
                   165                 170                 175
   TCA GGT GTC GGG GTC CCC ATC ATG GTC ATG GCA GTG ACC CAA CCC CGG     634
   Ser Gly Val Gly Val Pro Ile Met Val Met Ala Val Thr Gln Pro Arg
               180                 185                 190
   GAT GGT GCA GTG GTA TGC ATG CTC CAG TTC CCC AGT CCC AGC TGG TAC     682
   Asp Gly Ala Val Val Cys Met Leu Gln Phe Pro Ser Pro Ser Trp Tyr
           195                 200                 205
   TGG GAC ACT GTG ACC AAG ATC TGC GTG TTC CTC TTT GCC TTC GTG GTG     730
   Trp Asp Thr Val Thr Lys Ile Cys Val Phe Leu Phe Ala Phe Val Val
       210                 215                 220
   CCG ATC CTC ATC ATC ACG GTG TGC TAT GGC CTC ATG CTA CTG CGC CTG     778
   Pro Ile Leu Ile Ile Thr Val Cys Tyr Gly Leu Met Leu Leu Arg Leu
   225                 230                 235                 240
   CGC AGC GTG CGT CTG CTG TCC GGT TCC AAG GAG AAG GAC CGC AGC CTG     826
   Arg Ser Val Arg Leu Leu Ser Gly Ser Lys Glu Lys Asp Arg Ser Leu
                   245                 250                 255
   CGG CGC ATC ACG CGC ATG GTG CTG GTG GTG GTG GGC GCC TTC GTG GTG     874
   Arg Arg Ile Thr Arg Met Val Leu Val Val Val Gly Ala Phe Val Val
               260                 265                 270
   TGC TGG GCG CCC ATC CAC ATC TTC GTC ATC GTC TGG ACG CTG GTG GAC     922
   Cys Trp Ala Pro Ile His Ile Phe Val Ile Val Trp Thr Leu Val Asp
           275                 280                 285
   ATC AAT CGG CGC GAC CCA CTT GTG GTG GCC GCA CTG CAC CTG TGC ATT     970
   Ile Asn Arg Arg Asp Pro Leu Val Val Ala Ala Leu His Leu Cys Ile
       290                 295                 300
   GCG CTG GGC TAC GCC AAC AGC AGC CTC AAC CCG GTT CTC TAC GCC TTC   1018
   Ala Leu Gly Tyr Ala Asn Ser Ser Leu Asn Pro Val Leu Tyr Ala Phe
   305                 310                 315                 320
   CTG GAC GAG AAC TTC AAG CGC TGC TTC CGC CAG CTC TGT CGC ACG CCC   1066
   Leu Asp Glu Asn Phe Lys Arg Cys Phe Arg Gln Leu Cys Arg Thr Pro
                   325                 330                 335
   TGC GGC CGC CAA GAA CCC GGC AGT CTC CGT CGT CCC CGC CAG GCC ACC   1114
   Cys Gly Arg Gln Glu Pro Gly Ser Leu Arg Arg Pro Arg Gln Ala Thr
               340                 345                 350
   ACG CGT GAG CGT GTC ACT GCC TGC ACC CCC TCC GAC GGC CCG GGC GGT   1162
   Thr Arg Glu Arg Val Thr Ala Cys Thr Pro Ser Asp Gly Pro Gly Gly
           355                 360                 365
   GGC GCT GCC GCC TGACCTACCC GACCTTCCCC TTAAACGCCC CTCCCAAGTG       1214
   Gly Ala Ala Ala
       370
   AAGTGATCCA GAGGCCACAC CGAGCTCCCT GGGAGGCTGT GGCCACCACC AGGACAGCTA  1274
   GAATTGGGCC TGCACAGAGG GGAGGCCTCC TGTGGGGACG GGGCCTGAGG GATCAAAGGC  1334
   TCCAGGTTGG AACGGTGGGG GTGAGGAAGC AGAGCTGGTG ATTCCTAAAC TGTATCCATT  1394
   AGTAAGGCCT CTCCAATGGG ACAGAGCCTC CGCCTTGAGA TAACATCGGG TTCTGGCCTT  1454
   TTTGAACACC CAGCTCCAGT CCAAGACCCA AGGATTCCAG CTCCAGGAAC CAGGAGGGGC  1514
   AGTGATGGGG TCGATGATTT GGTTTGGCTG AGAGTCCCAG CATTTGTGTT ATGGGGAGGA  1574
   TCTCTCATCT TAGAGAAGAT AAGGGGACAG GGCATTCAGG CAAGGCAGCT TGGGGTTTGG  1634
   TCAGGAGATA AGCGCCCCCT TCCCTTGGGG GGAGGATAAG TGGGGGATGG TCAACGTTGG  1694
   AGAAGAGTCA AAGTTCTCAC CACCTTTCTA ACTACTCAGC TAAACTCGTT GAGGCTAGGG  1754
   CCAACGTGAC TTCTCTGTAG AGAGGATACA AGCCGGGCCT GATGGGGCAG GCCTGTGTAA  1814
   TCCCAGTCAT AGTGGAGGCT GAGGCTGGAA AATTAAGGAC CAACAGCCTG GGCAATTTAG  1874
   TGTCTCAAAA TAAAATGTAA AGAGGGCTGG GAATGTAGCT CAGTGGTAGG GTGTTTGTGT  1934
   GAGGCTCTGG GATCAATAAG ACAAAACAAC CAACCAACCA AAAACCTTCC AAACAACAAA  1994
   ACCAACCCTC AAACCAAAAA ACTATGTGGG TGTCTCTGAG TCTGGTTTGA AGAGAACCCG  2054
   CAGCCCTGTA TCCCTGTGGG GCTGTGGACA GTGGGCAGAA GCAGAGGCTC CCTGGATCCT  2114
   GAACAAGGGC CCCAAAAGCA AGTTCTAAAG GGACCCCTGA AACCGAGTAA GCCTTTGTGT  2174
   CAAGAAGTGG GAGTACAACC AGAAAGGTGG CTGAGTGCTT TAGAG                  2219
(2) INFORMATION POUR LA SEO ID NO: 2:
   (i) CARACTERISTIQUES DE LA SEQUENCE:
      (A) LONGUEUR: 372 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Glu Leu Val Pro Ser Ala Arg Ala Glu Leu Gln Ser Ser Pro Leu
   1               5                   10                  15
   Val Asn Leu Ser Asp Ala Phe Pro Ser Ala Phe Pro Ser Ala Gly Ala
               20                  25                  30
   Asn Ala Ser Gly Ser Pro Gly Ala Arg Ser Ala Ser Ser Leu Ala Leu
           35                  40                  45
   Ala Ile Ala Ile Thr Ala Leu Tyr Ser Ala Val Cys Ala Val Gly Leu
       50                  55                  60
   Leu Gly Asn Val Leu Val Met Phe Gly Ile Val Arg Tyr Thr Lys Leu
   65                  70                  75                  80
   Lys Thr Ala Thr Asn Ile Tyr Ile Phe Asn Leu Ala Leu Ala Asp Ala
                   85                  90                  95
   Leu Ala Thr Ser Thr Leu Pro Phe Gln Ser Ala Lys Tyr Leu Met Glu
               100                 105                 110
   Thr Trp Pro Phe Gly Glu Leu Leu Cys Lys Ala Val Leu Ser Ile Asp
           115                 120                 125
   Tyr Tyr Asn Met Phe Thr Ser Ile Phe Thr Leu Thr Met Met Ser Val
       130                 135                 140
   Asp Arg Tyr Ile Ala Val Cys His Pro Val Lys Ala Leu Asp Phe Arg
   145                 150                 155                 160
   Thr Pro Ala Lys Ala Lys Leu Ile Asn Ile Cys Ile Trp Val Leu Ala
                   165                 170                 175
   Ser Gly Val Gly Val Pro Ile Met Val Met Ala Val Thr Gln Pro Arg
               180                 185                 190
   Asp Gly Ala Val Val Cys Met Leu Gln Phe Pro Ser Pro Ser Trp Tyr
           195                 200                 205
   Trp Asp Thr Val Thr Lys Ile Cys Val Phe Leu Phe Ala Phe Val Val
       210                 215                 220
   Pro Ile Leu Ile Ile Thr Val Cys Tyr Gly Leu Met Leu Leu Arg Leu
   225                 230                 235                 240
   Arg Ser Val Arg Leu Leu Ser Gly Ser Lys Glu Lys Asp Arg Ser Leu
                   245                 250                 255
   Arg Arg Ile Thr Arg Met Val Leu Val Val Val Gly Ala Phe Val Val
               260                 265                 270
   Cys Trp Ala Pro Ile His Ile Phe Val Ile Val Trp Thr Leu Val Asp
           275                 280                 285
   Ile Asn Arg Arg Asp Pro Leu Val Val Ala Ala Leu His Leu Cys Ile
       290                 295                 300
   Ala Leu Gly Tyr Ala Asn Ser Ser Leu Asn Pro Val Leu Tyr Ala Phe
   305 310 315 320
   Leu Asp Glu Asn Phe Lys Arg Cys Phe Arg Gln Leu Cys Arg Thr Pro
   325 330 335
   Cys Gly Arg Gln Glu Pro Gly Ser Leu Arg Arg Pro Arg Gln Ala Thr
   340 345 350
   Thr Arg Glu Arg Val Thr Ala Cys Thr Pro Ser Asp Gly Pro Gly Gly
   355 360 365
   Gly Ala Ala Ala
   370
(2) INFORMATION POUR LA SEQ ID NO: 3:
   (i) CARACTERISTIQUES DE LA SEQUENCE:
      (A) LONGUEUR: 999 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Homo sapiens
   (ix) CARACTERISTIQUE ADDITIONELLE:
      (A) NOM/CLE: CDS
      (B) EMPLACEMENT: 1..999
      (D) AUTRES RENSEIGNEMENTS: /product= "Partie du Recepteur opiode delta humain"

**TABLE 1**

| Ligand | Préférence vis-à-vis des sous-types | PKᵢ | |
|---|---|---|---|
| | | Cos K56 | NG 108-15 |
| Bremazocin | k=µ=δ | 8.25 ± 0.12 | 8.75 |
| DADLE | δ=µ>>>k | 8.22 ± 0.15 | 8.70 |
| DPDPE | δ>>µ>>k | 7.73 ± 0.48 | 8.30 |
| Levorphanol | µ>>k=δ | 7.68 ± 0.07 | nd |
| Naloxone (-) | µ>δ=k | 7.53 ± 0.09 | nd |
| Etonitazen | µ>>>δ | 5.75 | 6 |
| DAGO | µ>>δ>k | 5.21 ± 0.17 | 5.65 |
| U 50488 | k>>>µ>δ | 4.35 ± 0.15 | 4.24 |

## Revendications

1. Séquence nucléotidique codant pour un polypeptide ayant une activité de récepteur opioïde delta choisie parmi :
(a) tout ou partie de la séquence nucléotidique SEQ ID N°3, ladite partie codant pour un polypeptide ayant une activité de récepteur opioïde et
(b) les séquences dérivées des séquences (a) en raison de la dégénérescence du code génétique.

2. Séquence nucléotidique complémentaire d'une séquence selon la revendication 1.

3. Séquence nucléotidique selon l'une des revendications 1 à 2, **caractérisée en ce qu'**il s'agit de séquences génomiques, d'ADNc, d'ARN, de séquences hybrides ou de séquences synthétiques ou semi-synthétiques.

4. Séquence nucléotidique **caractérisée en ce qu'**elle comprend toute la séquence nucléotidique SEQ ID N° 3 ou son brin complémentaire.

5. Séquence nucléotidique codant pour un récepteur humain opioïde δ homologue du récepteur opioïde δ de souris, ladite séquence étant susceptible d'être clonée par criblage d'une banque génomique humaine réalisée à partir de placenta, au moyen d'une sonde correspondant à la partie codante de la séquence SEQ ID N° 1 dans les conditions de stringence suivantes: 5XSSC, 5X Denhardt's, 40 % formamide, 0,1 % SDS, 0,05 % NaPPl, 100 µg/ml de sperme de saumon, 42°C.

6. Séquence nucléotidique selon la revendication 5, **caractérisée en ce qu'**elle comprend la séquence représentée dans la séquence SEQ ID N°3.

7. Séquence nucléotidique selon la revendication 6, **caractérisée en ce qu'**elle code pour un polypeptide ayant 372 acides aminés.

8. Polypeptide résultant de l'expression d'une séquence nucléotidique selon l'une des revendications 1 et 3 à 7.

9. Polypeptide comprenant toute la séquence peptidique SEQ ID N°3.

10. Polypeptide selon l'une des revendications 8 ou 9 ayant une activité de récepteur de type opioïde delta.

11. Polypeptide ayant une activité de récepteur opioïde delta, homologue au polypeptide SEQ ID N° 2, codé par une séquence nucléotidique susceptible d'être obtenue par criblage d'une banque génomique humaine réalisée à partir de placenta, au moyen d'une sonde correspondant à la partie codante de la séquence SEQ ID N° 1 et dans les conditions de stringence élevée suivantes : 5XSSC, 5X Denhardt's, 40 % formamide, 0,1 % SDS, 0,05 % NaPPI, 100 µg/ml de sperme de saumon, 42°C.

12. Séquence anti-sens constituée par une séquence nucléotidique selon l'une quelconque des revendications 1 à 7, capable d'inhiber au moins partiellement la production d'un polypeptide selon l'une des revendications 8 à 11.

13. Anticorps susceptible d'être généré en utilisant comme antigène un polypeptide selon l'une des revendications 8 à 11.

14. Cellule recombinée capable d'exprimer à sa surface un polypeptide selon l'une des revendications 8 à 11, ladite cellule n'exprimant pas naturellement ce polypeptide.

15. Cellule selon la revendication 14 **caractérisée en ce qu'**elle est choisie parmi les cellules eucaryotes ou procaryotes.

16. Procédé de mise en évidence et/ou d'isolement de ligands de récepteurs opioïdes **caractérisé en ce que** l'on réalise les étapes suivantes :
- on met en contact une molécule ou un mélange contenant différentes molécules, éventuellement non identifiées, avec une cellule recombinée selon l'une des revendications 14 ou 15 exprimant à sa surface un polypeptide opioïde, dans des conditions permettant l'interaction entre ledit polypeptide et ladite molécule dans le cas où celle-ci posséderait une affinité pour ledit polypeptide, et,
- on détecte et/ou isole les molécules liées au dit polypeptide.

17. Procédé selon la revendication 16 pour la mise en évidence et/ou l'isolement de ligands des récepteurs opioïdes delta.

18. Procédé de mise en évidence et/ou d'isolement de modulateurs des récepteurs opioïdes, **caractérisés en ce que** l'on réalise les étapes suivantes :
- on met en contact une molécule ou un mélange contenant différentes molécules, éventuellement non-identifiées, avec une cellule recombinée selon l'une des revendications 14 ou 15 exprimant à sa surface un polypeptide ayant une activité de récepteur opioïde, en présence d'un ligand dudit récepteur, dans des conditions permettant l'interaction entre ledit polypeptide et le ligand, et,
- on détecte et/ou isole les molécules capables de moduler l'activité du ligand sur ledit polypeptide.

19. Procédé selon la revendication 18 pour la mise en évidence et/ou l'isolement de modulateurs des récepteurs opioïdes delta.

## Patentansprüche

1. Nukleotidsequenz, die für ein Polypeptid mit Delta-Opioid-Rezeptoraktivität codiert, ausgewählt aus der Reihe:
(a) die ganze Nukleotidsequenz SEQ ID Nr. 3 oder ein Teil davon, wobei dieser Teil für ein Polypeptid mit Opioid-Rezeptoraktivität codiert, sowie
(b) die aufgrund des degenerierten genetischen Codes von den Sequenzen (a) abgeleiteten Sequenzen.

2. Nukleotidsequenz, die zu einer Sequenz nach Anspruch 1 komplementär ist.

3. Nukleotidsequenz nach Anspruch 1 oder 2, **dadurch gekennzeichnet, daß** es sich um genomische Sequenzen, cDNA-Sequenzen, RNA-Sequenzen, Hybridsequenzen oder synthetische bzw. halbsynthetische Sequenzen handelt.

4. Nukleotidsequenz, **dadurch gekennzeichnet, daß** sie die gesamte Nukleotidsequenz SEQ ID Nr. 3 oder ihren komplementären Strang umfaßt.

5. Nukleotidsequenz, die für einen menschlichen Delta-Opioid-Rezeptor, der zu dem Delta-Opioid-Rezeptor der Maus homolog ist, codiert, wobei diese Sequenz **dadurch** cloniert werden kann, daß man eine ausgehend von der Plazenta hergestellte menschliche Genombibliothek mit einer Sonde, die dem codierenden Teil der Sequenz SEQ ID Nr. 1 entspricht, unter den folgenden stringenten Bedingungen durchmustert: 5X SSC, 5X Denhardtsche Lösung, 40% Formamid, 0,1% SDS, 0,05% NaPPI, 100 µg/ml Lachssperma, 42°C.

6. Nukleotidsequenz nach Anspruch 5, **dadurch gekennzeichnet, daß** sie die in Sequenz SEQ ID Nr. 3 dargestellte Sequenz umfaßt.

7. Nukleotidsequenz nach Anspruch 6, **dadurch gekennzeichnet, daß** sie für ein Polypeptid mit 372 Aminosäuren codiert.

8. Polypeptid, das durch die Expression einer Nukleotidsequenz nach einem der Ansprüche 1 und 3 bis 7 entsteht.

9. Polypeptid, das die gesamte Peptidsequenz SEQ ID Nr. 3 umfaßt.

10. Polypeptid nach Anspruch 8 oder 9 mit einer Rezeptoraktivität des Delta-Opioid-Typs.

11. Polypeptid mit Delta-Opioid-Rezeptoraktivität, das zu dem Polypeptid SEQ ID Nr. 2, welches durch eine Nukleotidsequenz, die **dadurch** erhalten werden kann, daß man eine ausgehend von der Plazenta hergestellte menschliche Genombibliothek mit einer Sonde, die dem codierenden Teil der Sequenz SEQ ID Nr. 1 entspricht, unter den folgenden hochstringenten Bedingungen durchmustert: 5X SSC, 5X Denhardtsche Lösung, 40% Formamid, 0,1% SDS, 0,05% NaPPI, 100 µg/ml Lachssperma, 42°C, codiert wird, homolog ist.

12. Antisens-Sequenz die aus einer Nukleotidsequenz nach einem der Ansprüche 1 bis 7 besteht und die die Produktion eines Polypeptids nach einem der Ansprüche 8 bis 11 zumindest teilweise zu hemmen vermag.

13. Antikörper, der **dadurch** erhältlich ist, daß man ein Polypeptid nach einem der Ansprüche 8 bis 11 als Antigen verwendet.

14. Rekombinante Zelle, die ein Polypeptid nach einem der Ansprüche 8 bis 11 an ihrer Oberfläche zu exprimieren vermag, wobei die Zelle dieses Polypeptid nicht natürlicherweise exprimiert.

15. Zelle nach Anspruch 14, **dadurch gekennzeichnet, daß** sie aus den eukaryontischen oder prokaryontischen Zellen ausgewählt ist.

16. Verfahren zum Nachweis und/oder zur Isolierung von Opioid-Rezeptorliganden, **dadurch gekennzeichnet, daß** man die folgenden Schritte durchführt:
- man bringt ein Molekül oder eine Mischung, die unterschiedliche Moleküle, die ggf. nicht identifiziert sind, enthält, mit einer rekombinanten Zelle nach Anspruch 14 oder 15, die ein Opioid-Polypeptid an ihrer Oberfläche exprimiert, unter Bedingungen, die dann, wenn das Molekül eine Affinität für dieses Polypeptid aufweisen könnte, eine Wechselwirkung zwischen dem Polypeptid und dem Molekül gestatten, in Kontakt und
- man weist die Moleküle, die an das Polypeptid gebunden sind, nach und/oder isoliert sie.

17. Verfahren nach Anspruch 16 zum Nachweisen und/oder zur Isolierung von Delta-Opioid-Rezeptorliganden.

18. Verfahren zum Nachweis und/oder zur Isolierung von Opioid-Rezeptormodulatoren, **dadurch gekennzeichnet, daß** man die folgenden Schritte durchführt:
- man bringt ein Molekül oder eine Mischung, die unterschiedliche Moleküle, die ggf. nicht identifiziert sind, enthält, mit einer rekombinanten Zelle nach Anspruch 14 oder 15, die ein Polypeptid mit Opioid-Rezeptoraktivität an ihrer Oberfläche exprimiert, unter Bedingungen, die die Wechselwirkung zwischen dem Polypeptid und dem Liganden gestatten, in Gegenwart eines Liganden dieses Rezeptors in Kontakt und
- man weist die Moleküle, die die Aktivität des Liganden an dem Polypeptid zu modulieren vermögen, nach und/oder isoliert sie.

19. Verfahren nach Anspruch 18 zum Nachweis und/oder zur Isolierung von Modulatoren von Delta-Opioid-Rezeptoren.

## Claims

1. Nucleotide sequence which encodes a polypeptide having a delta opioid receptor activity, selected from among:
(a) all or part of the nucleotide sequence SEQ ID NO: 3, the said part encoding a polypeptide having an opioid receptor activity, and
(b) sequences derived from sequences (a) on account of the degeneracy of the genetic code.

2. Complementary nucleotide sequence of a sequence according to Claim 1.

3. Nucleotide sequence according to either of Claims 1 and 2, **characterized in that** it involves genomic cDNA or RNA sequences, hybrid sequences, or synthetic or semi-synthetic sequences.

4. Nucleotide sequence, **characterized in that** it comprises all of the nucleotide sequence SEQ ID NO: 3, or of its complementary strand.

5. Nucleotide sequence encoding a human δ opioid receptor which is homologous to the mouse δ opioid receptor, the said sequence being able to be cloned by screening a human genomic library prepared from placenta with a probe corresponding to the coding part of the sequence SEQ ID No: 1 under the following stringency conditions: 5 x SSC, 5 x Denhardt's, 40% formamide, 0.1% SDS, 0.05% NaPPI, 100 µg/ml salmon sperm, 42°C.

6. Nucleotide sequence according to Claim 5, **characterized in that** it comprises the sequence depicted in sequence SEQ ID No: 3.

7. Nucleotide sequence according to Claim 6, **characterized in that** it encodes a polypeptide having 372 amino acids.

8. Polypeptide which results from the expression of a nucleotide sequence according to any one of Claims 1 and 3 to 7.

9. Polypeptide which comprises all of the peptide sequence SEQ ID NO: 3.

10. Polypeptide according to either one of Claims 8 and 9 having a receptor activity of the delta opioid type.

11. Polypeptide which has a delta opioid receptor activity, which is homologous to the polypeptide SEQ ID No: 2 and which is encoded by a nucleotide sequence which can be obtained by screening a human genomic library prepared from placenta with a probe corresponding to the coding part of the sequence SEQ ID No: 1 and under the following high stringency conditions: 5 x SSC, 5 x Denhardt's, 40% formamide, 0.1% SDS, 0.05% NaPPI, 100 µg/ml salmon sperm, 42°C.

12. Antisense sequence consisting of a nucleotide sequence according to any one of Claims 1 to 7, which is able at least partially to inhibit production of a polypeptide according to one of Claims 8 to 11.

13. Antibody which can be generated using a polypeptide according to one of Claims 8 to 11 as antigen.

14. Recombinant cell which is able to express, at its surface, a polypeptide according to one of Claims 8, 11, with the said cell not naturally expressing this polypeptide.

15. Cell according to Claim 14, **characterized in that** it is selected from among eukaryotic or prokaryotic cells.

16. Procedure for detecting and/or isolating ligands of opioid receptors, **characterized in that** the following steps are carried out:
- a molecule, or a mixture containing different molecules, which may not have been identified, is brought into contact with a recombinant cell according to one of Claims 14 or 15, which cell expresses, at its surface, an opioid polypeptide, under conditions which permit interaction between the said polypeptide and the said molecule, if the latter possesses an affinity for the said polypeptide, and
- the molecules bound to the said polypeptide are detected and/or isolated.

17. Procedure according to Claim 16 for detecting and/or isolating ligands of delta opioid receptors.

18. Procedure for detecting and/or isolating modulators of opioid receptors, **characterized in that** the following steps are carried out:
- a molecule, or a mixture containing different molecules, which may not have been identified, is brought into contact with a recombinant cell according to one of Claims 14 or 15, which cell expresses, at its surface, a polypeptide having an opioid receptor activity, in the presence of a ligand of the said receptor, under conditions which permit interaction between the said polypeptide and the ligand, and
- the molecules which are capable of modulating the activity of the ligand on the said polypeptide are detected and/or isolated.

19. Procedure according to Claim 18 for detecting and/or isolating modulators of delta opioid receptors.
